# EUROPEAN PATENT SPECIFICATION

(11) **EP 4 262 625 B1**
(45) Date of publication and mention of the grant of the patent: **07.05.2025**
(21) Application number: 20838406.5
(22) Date of filing: 17.12.2020
(51) Int. Cl.: A61F 2/07, A61F 2/00

(54) **STENT GRAFT AND METHOD OF MAKING A STENT GRAFT**
STENTGRAFT UND VERFAHREN ZUR HERSTELLUNG EINES STENTGRAFTS
MISE EN PLACE D'UNE ENDOPROTHÈSE ET PROCÉDÉ DE MISE EN PLACE D'UNE ENDOPROTHÈSE

(43) Date of publication of application: 25.10.2023
(73) Proprietor: Angiomed GmbH & Co. Medizintechnik KG, 76227 Karlsruhe (DE)
(72) Inventor: KLODT, Katrin, 76227 Karlsruhe (DE); VOGEL, Michael, 76227 Karlsruhe (DE); SUPPER, Wolfgang, 76227 Karlsruhe (DE)
(74) Representative: Hoffmann Eitle
(86) International application number: PCT/EP2020/086716
(87) International publication number: WO 2022/128094

(56) References cited:
- WO-A1-00/59405
- US-A1- 2003 181 973

## Description

### TECHNICAL FIELD

The present invention relates to stent grafts and methods of making them.

### TECHNICAL BACKGROUND

Stent grafts, that is, covered stents, are frequently used in medicine in endovascular repairs and in a range of other procedures, such as in the placement of transjugular intrahepatic portosystemic shunts (TIPS). Compared with uncovered stents, which are often used for keeping occluded or otherwise restricted blood vessels open and have walls that can be easily penetrated by blood, stent grafts are by themselves tubular conduits which can, to some extent, replace the blood conducting function of a blood vessel.

A typical stent graft consists of three layers. There is firstly the base stent which is the stent that gives structural integrity to the stent graft and that defines its shape. Such base stents can be made of self-expanding materials such as nitinol that expands to a delivery configuration when implanted in a body due to being heated up to the patient's body temperature. It is also possible that the base stent as such is balloon expandable and can be expanded to its final delivery configuration by means of a balloon that is inserted into it and is then expanded.

In the prior art, stent grafts are often covered on their inside and on their outside with a covering layer that is made of a biocompatible lining material (e.g. ePTFE or FEP). This covering material turns the scaffolding provided for by the base stent into a tubular structure that can serve as a blood conduit.

With such stent grafts, a common problem is that one wants to improve the flexibility of the stent graft and to reduce its delivery profile. Put simply, having a higher degree of flexibility is advantageous in planting the stent graft in potentially tortuous vessels. Having a reduced delivery profile aids in being able to deliver it into narrow blood vessels and helps to reduce vessel trauma and patient discomfort. In prior art devices, these two aims have been achieved by building the outer covering layer not as a single integral layer but as a ribbon band arranged as a helix around an outer surface of the base stent or by reducing both layer thicknesses to some extent which all involved downsides during processing. Accordingly, since the effective wall thickness of the outer wall is reduced, the stent graft is more flexible and has a reduced delivery profile. However, with such stent grafts, one needs to ensure that the covering material is securely attached to the stent graft. Further, having a structure where a covering layer is provided both on the inside and the outside of the base stent can lead to wear, fretting and fatigue at the junction.

Increasing the adhesion of the covering material to the base stent is also a general point of concern. It is important to ensure that the covering material is securely attached to the base stent. If it is not, the stent graft is not suitable for implantation and needs to be discarded.

US 2003/181973 A1 discloses features falling under the preamble of claim 1. WO 00/59405 A1 is further prior art.

### SUMMARY OF THE INVENTION

The present invention aims at solving or alleviating one or more of the problems mentioned previously and in particular aims at providing a stent graft that is improved in the adherence of the covering material to the base stent whilst also making it possible to improve the flexibility and to reduce the delivery profile. The invention also aims at providing a method of making a stent graft having such advantages.

The present invention is defined by claim 1 and claim 7. Embodiments are defined in the dependent claims.

According to the invention, the stent graft comprises a base stent. This base stent, which could be a self-expanding or a balloon-expanding base stent, has a first and a second longitudinal end. A lumen extends longitudinally through the base stent between the first end and the second longitudinal end so that overall, the base stent is tubular.

A covering material is provided so as to line the base stent. This covering material turns the base stent into a stent graft that can function so as to conduct blood flowing therethrough. The covering material is fixed to the base stent so that the base stent and the covering material can be treated as one mechanical unit.

According to the invention, the base stent has a roughened surface that is at least partially penetrated by the covering material so as to form a positive fit. That is, there are recesses and/or protrusions formed by the material of the base stent with which the covering material engages and which aid in holding the covering material on the base stent. Having such a roughened surface thus leads to a better adhesion of the covering material to the base stent. By a roughened surface, it is meant that the surface is not smooth and is thus structured, with the structure being shaped so as to allow the covering material to penetrate into it. The roughened surface can be provided on all parts of the base stent - i.e. both the struts and the connectors.

In embodiments, the covering material is provided on the inside of the base stent only without covering the outside of the base stent. Whilst it cannot be excluded that small amounts of the covering material are pushed towards the outside of the base stent during production, the outside of the base stent is largely uncovered. Having such an arrangement, where the covering material is provided on the inside of the base stent only, improves both the flexibility of the stent graft and its delivery profile. This is because the cross-sectional area of the stent graft is reduced, which both increases the flexibility (due to a lower flexural rigidity) and also reduces the cross-section, which improves the delivery profile.

In the same way, in other embodiments, the covering material is provided on the outside of the base stent only without covering the inside of the base stent. Again, whilst it cannot be excluded that small amounts of the covering material are pushed towards the inside of the stent during production, the inside of the base stent is largely uncovered. Again, such a configuration improves the flexibility and the delivery profile. Further, compared with the configuration where the covering material is provided on the inside of the base stent only, such a stent graft is easier to manufacture since it will generally be easier to provide the microcavities for the adhesion of the covering material on the outside of the base stent, rather than the inside. For example, it is easier to sandblast a base stent from the outside, rather than the inside, to create those microcavities, and the same also holds for other ways of creating them like using a laser (which is easier to irradiate on the outer surface of the base stent).

In embodiments, the covering material forms a tube that, in some embodiments, extends along the whole length of the base stent. By having a tube that extends along the whole length of the base stent, no part of the base stent is uncovered. That is, there are no uncovered bits of the base stent that could otherwise interfere with the blood flow. Accordingly, the usable length of the base stent is maximized.

In embodiments, the roughened surface has been roughened by sandblasting. Such a rough surface is particularly advantageous when it comes to adhering the base stent. It is also comparatively easy to implement such a method.

In the invention, the roughened surface comprises microcavities. Those microcavities are particularly good when it comes to adhering the covering material. Such microcavities can be understood to be pores or holes in the surface at a lengthscale that is significantly less than the thickness of the components they are disposed in. For example, the lengthscale (diameter) of such unevenesses could be less than 50% of that thickness, preferably less than 20% of that thickness. In the invention, those micro cavities comprise blind holes. By having blind holes, the covering material does not have to penetrate through the whole of the base stent, which avoids weakening the covering material at those positions where it flows into the base stent (since the amount of material of covering material that flows into the holes is limited).

In embodiments, the stent graft comprises holes that penetrate through the entirety of the base stent material. Such penetrating holes, that is, holes, which extend from the inside of the base stent to the outside allow for the covering material to fully penetrate through the base stent and to reach the other surface. This leads to a particularly strong adhesion of the covering material to the base stent thanks to the material flowing through these holes leading to a rivet effect. Such penetrating holes can be also combined with blind holes (i.e., some of the holes of the base stent could be blind holes whilst other holes are penetrating holes).

In another aspect of the invention, a method of making a stent graft is provided. According to that method, a base stent is provided that has a first and a second longitudinal end and a lumen extending longitudinally therethrough. The stent has a rough surface facing the lumen.

Subsequently, a tubular covering material is arranged on the stent. It can be slid into the stent but it could also be slid onto the stent so that the covering material could line the inner lumen of the base stent or, at least in theory, also the periphery of the base stent. The tubular covering material is then made to at least partially penetrate into the rough surface so as to form a positive fit to thereby anchor the covering material to the base stent. In order to make it penetrate, a pressure is applied to the covering material so as to push it into the microcavities. For example, a ribbon can be tightly wound around the covering material to apply a pressure to it, or an inflatable balloon can be introduced into the lumen of the base stent and then expanded at that position. If the covering material softens when heated up, it is also generally advisable to warm up the covering material beyond the temperature where it softens. For a covering material such as ePTFE, a temperature of about 335°C, which is slightly above its melting point of 327°C has proven to lead to good results. With that method, a stent graft is provided that has an improved adhesion of the covering material to the base stent. If the covering material is only provided on the inside or on the outside of the base stent, the delivery profile and the flexibility of the manufactured stent graft are also improved.

In embodiments, the stent of providing the base stent comprises purposefully roughening the surface facing the lumen. That is, a previously smoother stent is roughened. This can improve the adhesion since one can choose a method of roughening the surface that leads to an improved penetration of the covering material. Of note, the roughening step can also be used on an already rough pre-fabricated base stent so that it does not imply that surface of the base stent prior to the roughening is smooth.

In embodiments, the step of roughening comprises one or more steps of micro grinding, press forming and/or sandblasting the surface facing the lumen. Those steps lead to a particularly good roughened surface.

In embodiments, the step of roughening comprises applying a laser to the surface to be roughened. With such a laser, one can in a very controlled manner roughen the surface. Since one can thereby have a good degree of control over how to roughen that surface, the resulting adhesion properties can be accurately adjusted.

In embodiments, the rough surface comprises blind holes into which the covering material penetrates at least partially. Such blind holes limit the amount of covering material that can penetrate into them. This avoids undue weakening of the covering material at those holes.

In embodiments, the rough surface comprises holes that penetrate through the entirety of the material of the base stent from the luminal side of the base stent to the abluminal side of the base stent. As mentioned before, the adhesion is improved with such a configuration.

### BRIEF DESCRIPTION OF THE DRAWINGS

Figure 1 shows a stent graft according to one embodiment of the invention.
Figure 2 shows a method of making a stent graft according to one embodiment of the invention.

### DETAILED DESCRIPTION OF THE DRAWINGS

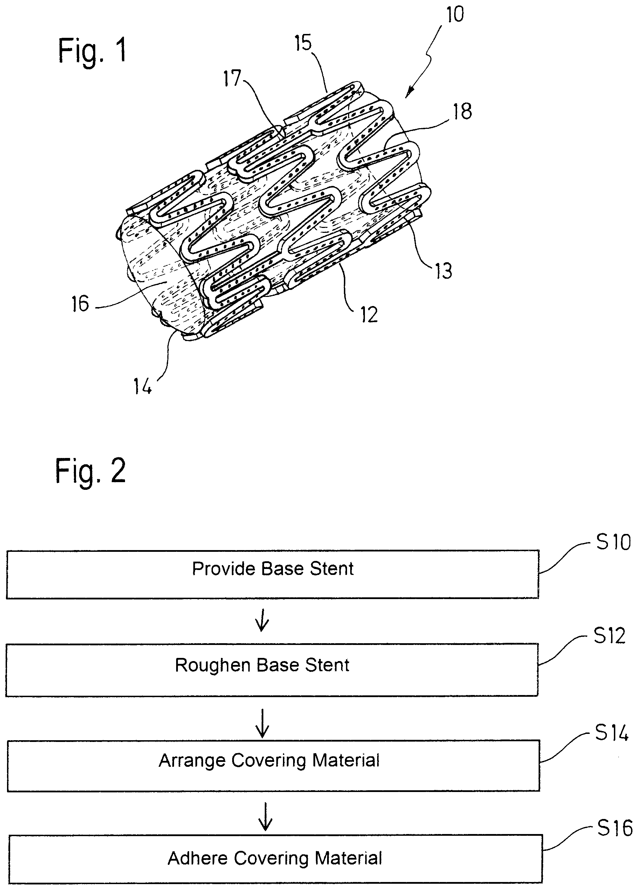

Figure 1 shows, in a perspective view, a stent graft according to an embodiment of the invention. A stent graft 10 is provided that comprises a base stent 12 having three rings 13 made of zigzagging struts 15. Those rings 13 are connected by means of connectors 17. Provided on the inside of the base stent 12 is an inner covering 14 that lines the lumen 16 of the stent graft 10.

As can be seen from the drawing, the base stent 12 furthermore comprises throughholes 18 that are arranged so as to radially extend from a luminal surface of the base stent 12 to an abluminal surface of the base stent 12 and that are at least partially penetrated by the covering material 14. The covering material, which could be ePTFE, flows into those holes during sintering and as a result creates an adhesion and mechanical grip between the stent graft 12 and the covering material 14. Throughholes 18 are provided both on the connectors 17 and the struts 15 of the rings 13.

Figure 2 shows in a flow chart a way of producing the stent graft 10 that is shown in Figure 1.

In step S10, a base stent 12 is provided.

Subsequently, in step S12, this base stent 12 is roughened. This roughening could be performed using micro grinding which delivers a higher roughness than pre-polished base stents (which is the standard surface treatment for such stents). It would also be an option to use a porous nitinol material prior to cutting the stent. Alternative ways would be to sandblast the base stent surface or to use some other mechanical surface treatment techniques such as pressing. Alternatively, laser micro cavities could be made, or one could use a laser to produce blind holes. One could also make throughholes 18 that completely cut through the base stent 12 from the luminal side to the abluminal side, as is shown in Figure 1. In the case of using a micro grinding and of using a porous nitinol material, steps S10 and S12 can be a single step so that no separate roughening step is needed beyond providing a sufficiently porous base stent 12.

In a subsequent step S14 of arranging the covering material 14, the covering material 14 is arranged on the base stent. It can be arranged in the lumen 16 of the base stent 12, or it can be arranged so as to surround the base stent.

Finally, through a sintering process similar to what is done in the prior art, the covering material 14 is adhered to the base stent 12 (step S16). In that step, in embodiments, one could use a high enough pressure so as to force the covering material 14 such as ePTFE to flow into the roughened surface and, if present, the holes in the base stent surface so as to increase the grip of the covering material 14. This improves the mechanical fixation and can, in the case of there being throughholes, lead to the covering material flowing through those throughholes 18 onto the opposite surface and thus to a particularly strong mechanical fixation of the covering material to the base stent. The adhesion step is otherwise similar to the adhesion step as performed during prior art stent graft manufacturing processes.

## Claims

1. Stent graft, comprising:
- a base stent (12) having a first and a second longitudinal end, a lumen (16) extending longitudinally through the base stent (12),
- a covering material (14) provided so as to line the base stent (12),
the base stent (12) having a roughened surface, wherein the roughened surface comprises microcavities that are blind holes,
**characterized by**
the roughened surface being at least partially penetrated by the covering material (14) so as to form a positive fit.

2. Stent graft according to claim 1, wherein the covering material (14) is provided on the inside of the base stent (12) without covering the outside of the base stent (12).

3. Stent graft according to claim 1, wherein the covering material (14) is provided on the outside of the base stent (12) without covering the inside of the base stent (12).

4. Stent graft according to one of the preceding claims, wherein the covering material (14) forms a tube that extends along the whole length of the base stent (12).

5. Stent graft according to one of the preceding claims, wherein the roughened surface has been roughened by sandblasting.

6. Stent graft according to one of the preceding claims, wherein the stent graft comprises holes (18) that penetrate through the base stent (12).

7. Method of making a stent graft, comprising:
- providing a base stent (12), the base stent (12) having a first and a second longitudinal end and a lumen (16) extending longitudinally therethrough, the base stent (12) having a rough surface facing the lumen (16), wherein the rough surface comprises microcavities that are blind holes,
**characterized by**
- arranging a tubular covering material (14) inside the base stent (12) and causing it to at least partially penetrate into the rough surface so as to form a positive fit.

8. Method of claim 7, wherein the step of providing a base stent (12) comprises roughening the surface to be penetrated by the covering material (14).

9. Method of claim 8, wherein the step of roughening comprises a step of micro grinding, press forming and/or sandblasting the surface to be roughened.

10. Method of claim 8 or 9, wherein the step of roughening comprises applying a laser to the surface to be roughened.

11. Method of one of claims 7 to 10, wherein the rough surface comprises throughholes (18) that penetrate through the base stent (12).

## Patentansprüche

1. Stentgraft, der Folgendes umfasst:
- einen Basisstent (12), der ein erstes und ein zweites längliches Ende aufweist, wobei sich ein Lumen (16) der Länge nach durch den Basisstent (12) erstreckt,
- ein Abdeckmaterial (14), das so bereitgestellt ist, dass es den Basisstent (12) auskleidet,
wobei der Basisstent (12) eine aufgeraute Oberfläche aufweist, wobei die aufgeraute Oberfläche Mikrohohlräume umfasst, die Sacklöcher sind,
**dadurch gekennzeichnet, dass**
die aufgeraute Oberfläche mindestens teilweise von dem Abdeckmaterial (14) durchdrungen wird, so dass ein Formschluss gebildet wird.

2. Stentgraft nach Anspruch 1, wobei das Abdeckmaterial (14) auf der Innenseite des Basisstents (12) bereitgestellt ist, ohne dabei die Außenseite des Basisstents (12) zu bedecken.

3. Stentgraft nach Anspruch 1, wobei das Abdeckmaterial (14) auf der Außenseite des Basisstents (12) bereitgestellt ist, ohne dabei die Innenseite des Basisstents (12) zu bedecken.

4. Stentgraft nach einem der vorhergehenden Ansprüche, wobei das Abdeckmaterial (14) einen Schlauch bildet, der sich entlang der gesamten Länge des Basisstents (12) erstreckt.

5. Stentgraft nach einem der vorhergehenden Ansprüche, wobei die aufgeraute Oberfläche durch Sandstrahlen aufgeraut wurde.

6. Stentgraft nach einem der vorhergehenden Ansprüche, wobei der Stentgraft Löcher (18) umfasst, die den Basisstent (12) durchdringen.

7. Verfahren zum Herstellen eines Stentgrafts, das Folgendes umfasst:
- Bereitstellen eines Basisstents (12), wobei der Basisstent (12) ein erstes und ein zweites längliches Ende sowie ein Lumen (16) aufweist, das sich der Länge nach durch diesen erstreckt, wobei der Basisstent (12) eine aufgeraute Oberfläche aufweist, die dem Lumen (16) zugewandt ist, wobei die raue Oberfläche Mikrohohlräume umfasst, die Sacklöcher sind,
**gekennzeichnet durch**
- das Anordnen eines schlauchförmigen Abdeckmaterials (14) innerhalb des Basisstents (12) und Veranlassen, dass es mindestens teilweise in die raue Oberfläche durchdringt, so dass ein Formschluss gebildet wird.

8. Verfahren nach Anspruch 7, wobei der Schritt des Bereitstellens eines Basisstents (12) das Aufrauen der Oberfläche umfasst, so dass diese von dem Abdeckmaterial (14) durchdrungen werden kann.

9. Verfahren nach Anspruch 8, wobei der Schritt des Aufrauens einen Schritt des Mikroschleifens, Pressformens und/oder Sandstrahlens der Oberfläche umfasst, so dass diese aufgeraut wird.

10. Verfahren nach Anspruch 8 oder 9, wobei der Schritt des Aufrauens das Anwenden eines Lasers auf der Oberfläche umfasst, so dass diese aufgeraut wird.

11. Verfahren nach einem der Ansprüche 7 bis 10, wobei die raue Oberfläche Durchgangslöcher (18) umfasst, die den Basisstent (12) durchdringen.

## Revendications

1. Endoprothèse, comprenant :
- une endoprothèse de base (12) ayant une première et une seconde extrémité longitudinale, une lumière (16) s'étendant longitudinalement à travers l'endoprothèse de base (12),
- un matériau couvrant (14) disposé de manière à revêtir l'endoprothèse de base (12),
l'endoprothèse de base (12) ayant une surface rugueuse,
dans laquelle la surface rugueuse comprend des microcavités qui sont des trous borgnes,
**caractérisée par**
la surface rugueuse étant au moins partiellement pénétrée par le matériau couvrant (14) de manière à former un ajustement positif.

2. Endoprothèse selon la revendication 1, dans laquelle le matériau couvrant (14) est disposé sur l'intérieur de l'endoprothèse de base (12) sans couvrir l'extérieur de l'endoprothèse de base (12).

3. Endoprothèse selon la revendication 1, dans laquelle le matériau couvrant (14) est disposé sur l'extérieur de l'endoprothèse de base (12) sans couvrir l'intérieur de l'endoprothèse de base (12).

4. Endoprothèse selon l'une des revendications précédentes, dans laquelle le matériau couvrant (14) forme un tube qui s'étend sur toute la longueur de l'endoprothèse de base (12).

5. Endoprothèse selon l'une des revendications précédentes, dans laquelle la surface rugueuse a été rendue rugueuse par sablage.

6. Endoprothèse selon l'une des revendications précédentes, dans laquelle l'endoprothèse comprend des trous (18) qui pénètrent à travers l'endoprothèse de base (12).

7. Procédé de fabrication d'une endoprothèse, comprenant :
- la fourniture d'une endoprothèse de base (12), l'endoprothèse de base (12) ayant une première et une seconde extrémité longitudinale et une lumière (16) s'étendant longitudinalement à travers celle-ci, l'endoprothèse de base (12) ayant une surface rugueuse faisant face à la lumière (16), dans lequel la surface rugueuse comprend des microcavités qui sont des trous borgnes,
**caractérisé par**
- l'agencement d'un matériau couvrant tubulaire (14) à l'intérieur de l'endoprothèse de base (12) et le fait de l'amener à pénétrer au moins partiellement dans la surface rugueuse de manière à former une complémentarité de forme.

8. Procédé selon la revendication 7, dans lequel l'étape de fourniture d'une endoprothèse de base (12) comprend la rugosification de la surface que doit pénétrer le matériau couvrant (14).

9. Procédé selon la revendication 8, dans lequel l'étape de rugosification comprend une étape de micro-meulage, de formage à la presse et/ou de sablage de la surface à rendre rugueuse.

10. Procédé selon la revendication 8 ou la revendication 9, dans lequel l'étape de rugosification comprend l'application d'un laser sur la surface à rendre rugueuse.

11. Procédé selon l'une des revendications 7 à 10, dans lequel la surface rugueuse comprend des trous traversants (18) qui pénètrent à travers l'endoprothèse de base (12).
